# EUROPEAN PATENT SPECIFICATION

(11) **EP 0 322 691 B1**
(45) Date of publication and mention of the grant of the patent: **15.06.1994**
(21) Application number: 88121187.4
(22) Date of filing: 17.12.1988
(51) Int. Cl.: C07D 231/06, C07D 401/06, C07D 403/06, C07D 405/06, C07D 405/14

(54) **Process for producing 1-acyl-2-pyrazoline derivatives**
Verfahren zur Herstellung von 1-Acyl-2-pyrazolin-Derivaten
Procédé de préparation de dérivés d'acyl-1-pyrazoline 2

(30) Priority: 17.12.1987 JP 317446/87
(43) Date of publication of application: 05.07.1989
(73) Proprietor: MITSUI TOATSU CHEMICALS, Inc., Chiyoda-Ku Tokyo 100 (JP)
(72) Inventor: Yamashita, Hiroyuki, Oomuta-shi, Fukuoka-ken (JP); Okumura, Kunio, Kamakura-shi, Kanagawa-ken (JP); Iizuka, Hajime, Hiratsuka-shi, Kanagawa-ken (JP); Ohto, Norio, Sakae-ku,Yokohama-shi, Kanagawa-ken (JP)
(74) Representative: Thomsen, Dieter, Dr.

(56) References cited:
- EP-A- 0 035 792
- EP-A- 0 240 001

## Description

The invention relates to a process for the production of 1-acyl-2-pyrazoline derivatives which compounds have anti-cerebral edema activity, and thus these compounds are useful as medicaments and/or as intermediates e.g. in this field.

2-Pyrazoline derivatives having an acyl group in position 1 can be prepared by reacting a corresponding 2-pyrazoline derivative with an acid chloride or acid anhydride. EP 295 695-A2 describes certain 1-acyl-2-pyrazoline derivatives having anti-cerebral edema activity, i.e. 2-pyrazoline derivatives represented by the following formula 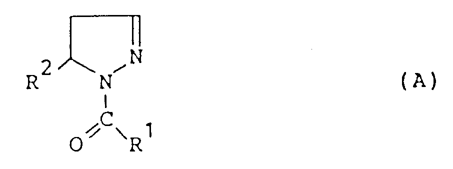
wherein R¹ denotes a pyridyl, a pyrazyl or an C₁₋₄-alkoxyl group; and R² stands for a hydrogen atom, an C₁₋₄-alkyl, a pyridyl, a furyl, a phenyl, or a C₁₋₄-alkyl substituted phenyl, a C₁₋₃-alkoxyl substituted phenyl or a halogen substituted phenyl group. The process for the production of such compounds uses several steps, which might be simplified, and gives also intermediates which are sensitive to oxygen (or air); thus by simplification of such a process it should also be possible to reduce the consumption of chemicals which are partly necessary in large excess.

EP 035 792-A2 is concerned with 1-acyl-2-pyrazolines which are prepared by reacting a pyrazoline derivative with an acid chloride; the method to get the final product is a complicated multistep production with the risk in the several stages of loss of intermediates and desired products by oxidation, isomerization, residue formation, by-products etc.

EP 240 001-A1 describes the production of 2-pyrazoline-5-ones by cyclization of substituted hydrazone derivatives in the presence of a base. The hydrazone cyclization uses the activated C=N-double bond (at the one N-atom) and the (with respect to the other N-atom) γ-position carbonyl-C atom of the 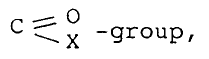
thus cleaving or setting free HX which reaction runs in the a.m. presence of a base; see reaction schema below.

Thus the object of the present invention is to create a production process for the 1-acyl-2-pyrazoline compounds which process is more simplified and leads to improved yields and more pure products by avoiding unstable intermediates and undesirable by-products.

According to the invention this object is solved by the process of claim 1, i.e. for the production of 1-acyl-2-pyrazoline derivatives of the formula 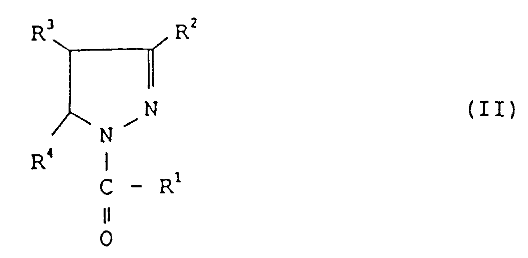
wherein R¹ denotes a hydrogen atom, a pyridyl group, a pyrazinyl group, a C₁-C₄-alkyl group, an unsubstituted phenyl group or a phenyl group substituted with one or more C₁-C₄-alkyl groups and/or one or more halogen atoms, or a C₁-C₄-alkoxy group; and R², R³ and R⁴ independently denote a hydrogen atom, a furyl group, a pyridyl group, a C₁-C₄-alkyl group or an unsubstituted phenyl group or a phenyl group substituted with one or more C₁-C₄-alkyl groups and/or one or more halogen atoms, which comprises cyclization at a temperature of from about 150 to about 300°C by heating of an acylhydrazone derivative of the formula 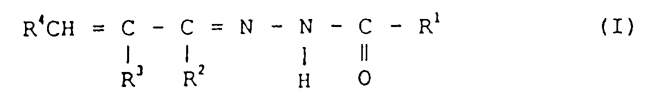
where R¹, R², R³ and R⁴ are defined as above.

The cyclization is conducted at a temperature of from preferably about 200 to about 250°C.

When practising the present invention it is found that the heat-induced cyclization of an acylhydrazone derivative gives advantageous results. The cyclization runs exactly; the yields are high; the products are pure. The inventive process uses as starting materials acylhydrazone derivatives of the formula 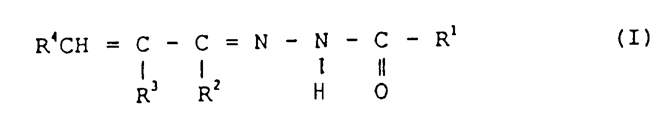
with the definitions for R¹, R², R³, R⁴ as given above. The inventive cyclization schema is 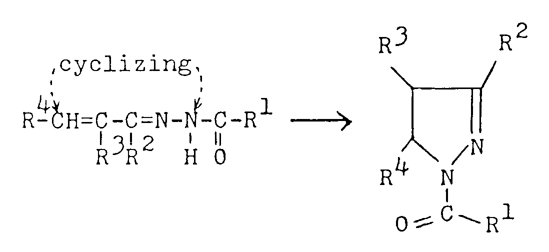
Quite contrarily on the other hand, the cyclization schema of the a.m. EP 240 001-A1 is as follows 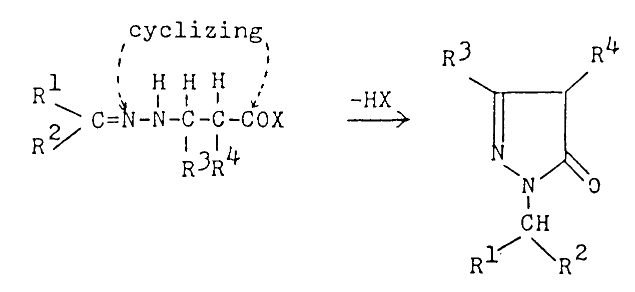
Examples of the acylhydrazone derivative include cinnamaldehyde benzoylhydrazone, cinnamaldehyde acetylhydrazone, cinnamaldehyde ethoxycarbonylhydrazone, cinnamaldehyde nicotinoylhydrazone, cinnamaldehyde 2-furylcarbonylhydrazone, o-methoxycinnamaldehyde nicotinoylhydrazone, crotonaldeyde nicotinoylhydrazone, crotonaldehyde pyrazinylcarbonylhydrazone, crotonaldehyde isonicotinoylhydrazone, crotonaldehyde picolinoylhydrazone, acrolein nicotinoylhydrazone, benzalacetone acetylhydrazone, 2-furylaldehyde nicotinoylhydrazone, 2-methylcinnamaldehyde nicotinoylhydrazone, benzal-2-furylmethylketone acetylhydrazone, 2-phenylcinnamaldehyde acetylhydrazone, and 2-methylaldehyde formylhydrazone.

These compounds (I) can be readily obtained through the condensation under dehydration of an acylhydrazine of the formula 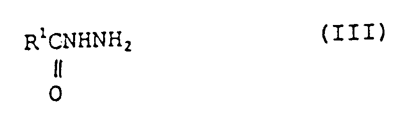
where R¹ denotes a hydrogen atom, a pyridyl group, a pyrazinyl group, a C₁-C₄-alkyl group, an aryl group or a C₁-C₄-alkoxy group, with an α, β-unsaturated carbonyl compound of the formula 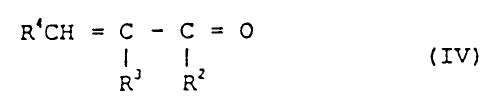
where R², R³ and R⁴ independently denote a hydrogen atom, a furyl group, a pyridyl group, a C₁-C₄-alkyl group or an aryl group. The acylhydrazine (III) and the α, β-unsaturated carbonyl compound (IV) are mixed and heated at 150 to 300°C in an inert solvent or without using any solvent. The acylhydrazone derivative (I) thus obtained can be reacted into the 1-acyl-2-pyrazoline derivative (II) by cyclization, i.e. without being isolated from the reaction liquid. The cyclizing reaction of the present invention is performed in the presence or absence of a solvent. When a solvent is used it can be an inert solvent, such as dichlorobenzene, naphthalene, biphenyl, quinoline, bibenzyl, diphenyl ether, diglyme or nitrobenzene. The solvent can be used in any amount necessary to carry out the reaction effectively. The amount of the solvent is usually 2 to 20 times the amount of the starting material.

The reaction pressure is selected according to the kind of solvent used; usually the reaction pressures are in the range of atmospheric pressure to about 50 kg/cm² = 50 bar .

The reaction may be carried out under any atmosphere; but a nitrogen atmosphere is preferable because it prevents the discoloration of the reaction liquid.

After the above-mentioned reactions, the desired product can be isolated by recrystallization or column chromatography in the usual way.

The inventive process permits the synthesis of 1-acyl-2-pyrazoline derivatives having anti-cerebral edema activity by the simple heating and cyclization of an acylhydrazone derivative;
this process is suitable for industrial production in large quantities.

The invention will be described in more detail with reference to the following examples.

### Example 1

### Synthesis of 1-benzoyl-5-phenyl-2-pyrazoline

To 200 ml of methanol solution containing 13.2 g of cinnamaldehyde was added 13.6 g of benzoylhydrazine, followed by stirring for 30 minutes at room temperature, for the precipitation of crystals. The crystals were filtered off and dried. Thus there was obtained 22 g of cinnamaldehyde benzoyl hydrazone (mp. 192-194°C).

Five grams of the cinnamaldehyde benzoylhydrazone was suspended in 20 ml of diphenyl ether, followed by stirring at 220°C for 3 hours. After cooling, the reaction liquid was poured into 100 ml of hexane for the separation of insoluble matter. The insoluble matter was filtered off and recrystallized from a methanol-water solution. Thus there was obtained 3.8 g of 1-benzoyl-5-phenyl-2-pyrazoline (mp. 105-106°C) as the desired product. The yield was 76.0%.

### Example 2

### Synthesis of 1-acetyl-5-phenyl-2-pyrazoline

To 10 ml of diphenyl ether were added 1.3 g of cinnamaldehyde and 0.8 g of acetylhydrazine, followed by gradual heating to 220°C over 30 minutes. Stirring was continued for 3 hours at that temperature. After cooling, the reaction liquid as such was subjected to silica gel chromatography (chloroform/methanol = 100/1). Thus there was obtained 1.2 g of 1-acetyl-5-phenyl-2-pyrazoline in the form of light yellowish oil as the desired product. The yield was 64.8%.

### Example 3

### Synthesis of 1-nicotinoyl-5-methyl-2-pyrazoline

In 50 ml of methanol was suspended 22 g of nicotinic acid hydrazide. To the suspension was added dropwise 13.2 ml of crotonaldehyde, followed by stirring for 30 minutes at room temperature. Methanol was distilled away under reduced pressure, and the residue washed with ether, filtered off, and dried. Thus there was obtained 27.5 g of crotonaldehyde nicotinoyl hydrazone (mp. 144-145°C).

Nineteen grams of this hydrazone was suspended in 50 ml of biphenyl ether, followed by stirring at 200-220°C (bath temperature) for 3 hours. After cooling, the reaction liquid was purified by silica gel chromatography (CHCl³:MeOH = 100:1). Thus there was obtained 8.0 g of 1-nicotinoyl-5-methyl-2-pyrazoline in the form of an oil (bp. 143-145°C/ 40 Pa (0,3 mm Hg)). The yield was 42.1%.

### Example 4

### Synthesis of 1-pyrazinylcarbonyl-5-methyl-2-pyrazoline

In 50 ml of methanol was suspended 9 g of nicotinic acid hydrazide. To the suspension was added dropwise 5.4 ml of crotonaldehyde, with stirring over 30 minutes at room temperature.

Methanol was distilled away under reduced pressure, and the residue washed with ether, filtered off, and dried. Thus there was obtained 11.2 g of crotonaldehyde pyrazinylcarbonyl hydrazone (mp. 192-194°C).

A quantity of 3.8 g of this hydrazone was suspended in 10 ml of diphenyl ether, followed by stirring at 200-220°C (bath temperature) for 3 hours. After cooling, the reaction liquid was purified by silica gel column chromatography (ethyl acetate). Thus there was obtained 1.4 g of 1-pyrazinylcarbonyl-5-methyl-2-pyrazoline (mp. 77-79°C). The yield was 36.8%.

### Examples 5 to 12

The same procedures as in Examples 1 to 4 were repeated to give the following compounds
· 1-ethoxycarbonyl-5-phenyl-2-pyrazoline (yield: 67%), oily substance
· 1-nicotinoyl-5-phenyl-2-pyrazoline (yield: 82%), mp. 100∼102°C
· 1-(2-furylcarbonyl)-5-phenyl-2-pyrazoline (yield: 51%), mp. 136∼137°C
· 1-nicotinoyl-5-(2-methoxyphenyl)-2-pyrazoline (yield: 71%), mp. 93∼95°C
· 1-nicotinoyl-2-pyrazoline (yield: 78%), mp. 81∼82°C
· 1-acetyl-3-methyl-5-phenyl-2-pyrazoline (yield: 66%), oily substance
· 1-nicotinoyl-5-(2-furyl)-2-pyrazoline (yield: 48%), mp. 91∼93°C
· 1-nicotinoyl-4-methyl-5-phenyl-2-pyrazoline (yield: 52%), oily substance

## Claims

1. Process for the production of 1-acyl-2-pyrazoline derivatives of the formula 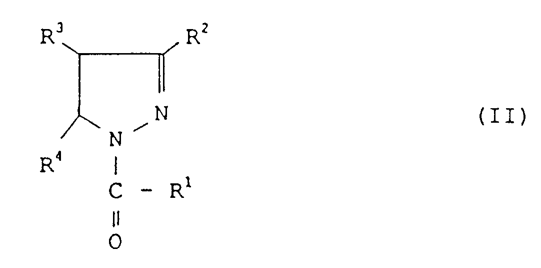 wherein R¹ denotes a hydrogen atom, a pyridyl group, a pyrazinyl group, a C₁-C₄-alkyl group, an unsubstituted phenyl group or a phenyl group substituted with one or more C₁-C₄-alkyl groups and/or one or more halogen atoms, or a C₁-C₄-alkoxy group; and R², R³ and R⁴ independently denote a hydrogen atom, a furyl group, a pyridyl group, a C₁-C₄-alkyl group or an unsubstituted phenyl group or a phenyl group substituted with one or more C₁-C₄-alkyl groups and/or one or more halogen atoms, which comprises cyclization at a temperature of from about 150 to about 300°C by heating of an acylhydrazone derivative of the formula 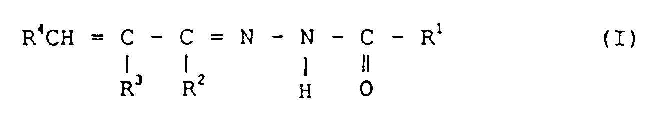 where R¹, R², R³ and R⁴ are defined as above.

2. Process according to the preceding claim wherein the cyclization is conducted at about 200 to about 250°C.

3. Process according to claim 1 wherein the acylhydrazone derivative is selected from the group consisting of cinnamaldehyde benzoylhydrazone, cinnamaldehyde acetylhydrazone, cinnamaldehyde ethoxycarbonylhydrazone, cinnamaldehyde nicotinoylhydrazone, cinnamaldehyde 2-furylcarbonylhydrazone, o-methoxycinnamaldehyde nicotinoylhydrazone, crotonaldehyde nicotinoylhydrazone, crotonaldehyde pyrazinylcarbonylhydrazone, crotonaldehyde isonicotinoylhydrazone, crotonaldehyde picolinoylhydrazone, acrolein nicotinoylhydrazone, benzalacetone acetylhydrazone, 2-furylaldehyde nicotinoylhydrazone, 2-methylcinnamaldehyde nicotinoylhydrazone, benzal-2-furylmethylketone acetylhydrazone, 2-phenylcinnamaldehyde acetylhydrazone, and 2-methylaldehyde formylhydrazone.

4. Process according to claim 1 wherein the compound prepared is 1-benzoyl-5-phenyl-2-pyrazoline.

5. Process according to claim 1 wherein the compound prepared is 1-acetyl-5-phenyl-2-pyrazoline.

6. Process according to claim 1 wherein the compound prepared is 1-nicotinoyl-5-methyl-2-pyrazoline.

7. Process according to claim 1 wherein the compound prepared is 1-pyrazinylcarbonyl-5-methyl-5-pyrazoline.

8. Process according to claim 1 wherein the compound prepared is 1-ethoxycarbonyl-5-phenyl-2-pyrazoline.

9. Process according to claim 1 wherein the compound prepared is 1-nicotinoyl-5-phenyl-2 pyrazoline.

10. Process according to claim 1 wherein the compound prepared is 1-(2-furylcarbonyl)-5-phenyl-2-pyrazoline.

11. Process according to claim 1 wherein the compound prepared is 1-nicotinoyl-5-(2-methoxyphenyl)-2-pyrazoline.

12. Process according to claim 1 wherein the compound prepared is 1-nicotinoyl-2-pyrazoline.

13. Process according to claim 1 wherein the compound prepared is 1-acetyl-3-methyl-5-phenyl-2-pyrazoline.

14. Process according to claim 1 wherein the compound prepared is 1-nicotinoyl-5-(2-furyl)-2-pyrazoline.

15. Process according to claim 1 wherein the compound prepared is 1-nicotinoyl-4-methyl-5-phenyl-2-pyrazoline.

## Patentansprüche

1. Verfahren zur Herstellung von 1-Acyl-2-pyrazolin-Derivaten der Formel 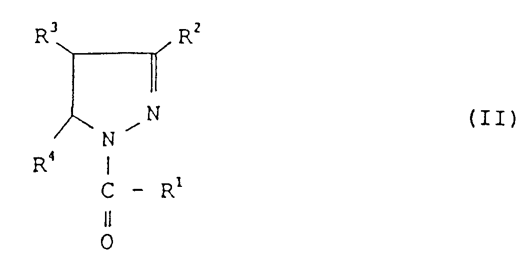 worin R¹ ein Wasserstoff-Atom, eine Pyridyl-Gruppe, eine Pyrazinyl-Gruppe, eine C₁-C₄-Alkyl-Gruppe, eine unsubstituierte Phenyl-Gruppe oder eine Phenyl-Gruppe, substituiert mit einer oder mehreren C₁-C₄-Alkyl-Gruppen und/oder einem oder mehreren Halogen-Atomen, oder eine C₁-C₄-Alkoxy-Gruppe bezeichnet; und R², R³ und R⁴ unabhängig voneinander ein Wasserstoff-Atom, eine Furyl-Gruppe, eine Pyridyl-Gruppe, eine C₁-C₄-Alkyl-Gruppe oder eine unsubstituierte Phenyl-Gruppe oder eine Phenyl-Gruppe, substituiert mit einer oder mehreren C₁-C₄-Alkyl-Gruppen und/oder einem oder mehreren Halogen-Atomen, bezeichnen, gekennzeichnet durch Cyclisierung bei einer Temperatur von etwa 150 bis etwa 300°C mittels Erhitzen eines Acylhydrazon-Derivats der Formel 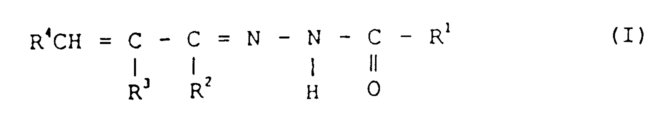 worin R¹, R², R³ und R⁴ die vorstehend angegebene Bedeutung haben.

2. Verfahren nach dem vorhergehenden Anspruch, worin die Cyclisierung bei etwa 200 bis etwa 250°C ausgeführt wird.

3. Verfahren nach Anspruch 1, worin das Acylhydrazon-Derivat aus der Reihe Cinnamaldehyd-Benzoylhydrazon, Cinnamaldehyd-Acetylhydrazon, Cinnamaldehyd-Ethoxycarbonylhydrazon, Cinnamaldehyd-Nicotinoylhydrazon, Cinnamaldehyd-2-Furylcarbonylhydrazon, o-Methoxycinnamaldehyd-Nicotinoylhydrazon, Crotonaldehyd-Nicotinoylhydrazon, Crotonaldehyd-Pyrazinylcarbonylhydrazon, Crotonaldehyd-Isonicotinoylhydrazon, Crotonaldehyd-Picolinoylhydrazon, Acrolein-Nicotinoylhydrazon, Benzalaceton-Acetylhydrazon, 2-Furylaldehyd-Nicotinoylhydrazon, 2-Methylcinnamaldehyd-Nicotinoylhydrazon, Benzal-2-furylmethylketon-Acetylhydrazon, 2-Phenylcinnamaldehyd-Acetylhydrazon und 2-Methylaldehyd-Formylhydrazon ausgewählt ist.

4. Verfahren nach Anspruch 1, worin die hergestellte Verbindung 1-Benzoyl-5-phenyl-2-pyrazolin ist.

5. Verfahren nach Anspruch 1, worin die hergestellte Verbindung 1-Acetyl-5-phenyl-2-pyrazolin ist.

6. Verfahren nach Anspruch 1, worin die hergestellte Verbindung 1-Nicotinoyl-5-methyl-2-pyrazolin ist.

7. Verfahren nach Anspruch 1, worin die hergestellte Verbindung 1-Pyrazinylcarbonyl-5-methyl-5-pyrazolin ist.

8. Verfahren nach Anspruch 1, worin die hergestellte Verbindung 1-Ethoxycarbonyl-5-phenyl-2-pyrazolin ist.

9. Verfahren nach Anspruch 1, worin die hergestellte Verbindung 1-Nicotinoyl-5-phenyl-2-pyrazolin ist.

10. Verfahren nach Anspruch 1, worin die hergestellte Verbindung 1-(2-Furylcarbonyl)-5-phenyl-2-pyrazolin ist.

11. Verfahren nach Anspruch 1, worin die hergestellte Verbindung 1-Nicotinoyl-5-(2-methoxyphenyl)-2-pyrazolin ist.

12. Verfahren nach Anspruch 1, worin die hergestellte Verbindung 1-Nicotinoyl-2-pyrazolin ist.

13. Verfahren nach Anspruch 1, worin die hergestellte Verbindung 1-Acetyl-3-methyl-5-phenyl-2-pyrazolin ist.

14. Verfahren nach Anspruch 1, worin die hergestellte Verbindung 1-Nicotinoyl-5-(2-furyl)-2-pyrazolin ist.

15. Verfahren nach Anspruch 1, worin die hergestellte Verbindung 1-Nicotinoyl-4-methyl-5-phenyl-2-pyrazolin ist.

## Revendications

1. Procédé de préparation de dérivés de la 1-acyl-2-pyrazoline répondant à la formule 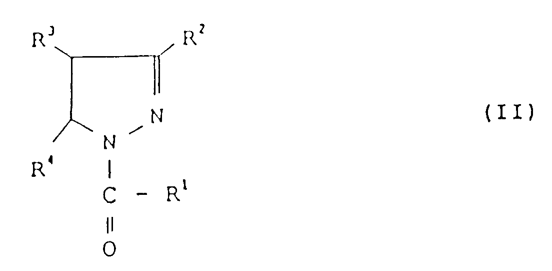 dans laquelle R¹ désigne un atome d'hydrogène, un groupe pyridyle, un groupe pyrazinyle, un groupe alkyle en C₁-C₄, un groupe phényle non substitué ou un groupe phényle substitué par un ou plusieurs groupes alkyle en C₁-C₄ et/ou un ou plusieurs atomes d'halogène, ou par un groupe alcoxy en C₁-C₄ ; et R², R³ et R⁴ désignent indépendamment un atome d'hydrogène, un groupe furyle, un groupe pyridyle, un groupe alkyle en C₁-C₄ ou un groupe phényle non substitué, ou un groupe phényle substitué par un ou plusieurs groupes alkyle en C₁-C₄ et/ou un ou plusieurs atomes d'halogène, qui comprend une cyclisation à une température d'environ 150 à environ 300°C par chauffage d'un dérivé de l'acide hydrazone répondant à la formule 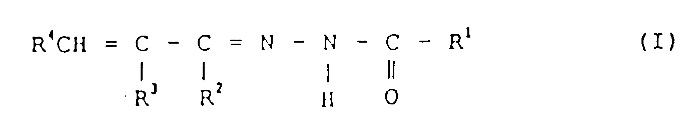 dans laquelle R¹, R², R³ et R⁴ sont tels que définis ci-dessus

2. Procédé selon la revendication précédente, dans lequel la cyclisation est effectuée à environ 200 à environ 250°C.

3. Procédé selon la revendication 1, dans lequel le dérivé de l'acide hydrazone est choisi parmi la cinnamaldéhyde benzoylhydrazone, la cinnamaldéhyde acétylhydrazone, de la cinnamaldéhyde éthoxycarbonylhydrazone, la cinnamaldéhyde nicotinoylhydrazone, la cinnamaldéhyde 2-furylcarbonylhydrazone, l'o-méthoxycinnamaldéhyde nicotinoylhydrazone, la crotonaldéhyde nicotinoylhydrazone, la crotonaldéhyde pyrazinylcarbonylhydrazone, la crotonaldéhyde isonicotinoylhydrazone, la crotonaldéhyde picolinoylhydrazone, l'acroléine nicotinoylhydrazone, la benzalacétone acétylhydrazone, la 2-furylaldéhyde nicotinoylhydrazone, la 2-méthylcinnamaldéhyde nicotinoylhydrazone, la benzal-2-furylméthylcétone acétylhydrazone, la 2-phénylcinnamaldéhyde acétylhydrazone, et la 2-méthyl-aldéhyde formylhydrazone.

4. Procédé selon la revendication 1, dans lequel le composé préparé est la 1-benzoyl-5-phényl-2-pyrazoline.

5. Procédé selon la revendication 1, dans lequel le composé préparé est la 1-acétyl-5-phényl-2-pyrazoline.

6. Procédé selon la revendication 1, dans lequel le composé préparé est la 1-nicotinoyl-5-méthyl-2-pyrazoline.

7. Procédé selon la revendication 1, dans lequel le composé préparé est la 1-pyrazinylcarbonyl-5-méthyl-5-pyrazoline.

8. Procédé selon la revendication 1, dans lequel le composé préparé est la 1-éthoxycarbonyl-5-phényl-2-pyrazoline.

9. Procédé selon la revendication 1, dans lequel le composé préparé est la 1-nicotinoyl-5-phényl-2-pyrazoline.

10. Procédé selon la revendication 1, dans lequel le composé préparé est la 1-(2-furylcarbonyl)-5-phényl-2-pyrazoline.

11. Procédé selon la revendication 1, dans lequel le composé préparé est la 1-nicotinoyl-5-(2-méthoxyphényl)-2-pyrazoline.

12. Procédé selon la revendication 1, dans lequel le composé préparé est la 1-nicotinoyl-2-pyrazoline.

13. Procédé selon la revendication 1, dans lequel le composé préparé est la 1-acétyl-3-méthyl-5-phényl-2-pyrazoline.

14. Procédé selon la revendication 1, dans lequel le composé préparé est la 1-nicotinoyl-5-(2-furyl)-2-pyrazoline.

15. Procédé selon la revendication 1, dans lequel le composé préparé est la 1-nicotinoyl-4-méthyl-5-phényl-2-pyrazoline.
